# EUROPEAN PATENT APPLICATION

(11) **EP 4 282 380 A1**
(43) Date of publication of application: **29.11.2023**
(21) Application number: 21791242.7
(22) Date of filing: 18.10.2021
(51) Int. Cl.: A61F 2/00, A61M 25/00

(54) **DRUG LEAKAGE PREVENTION DEVICE**

(30) Priority: 07.10.2021 KR 20210133145
(71) Applicant: Uroall, Gimhae-si, Gyeongsangnam-do 50969 (KR)
(72) Inventor: PARK, Myungchan, Busan 48107 (KR)
(74) Representative: Caspary, Karsten
(86) International application number: PCT/KR2021/014512
(87) International publication number: WO 2023/058805

(57) **Abstract**

A drug outflow preventing device is provided. A drug outflow preventing device according to an embodiment of the present invention is a drug outflow preventing device into which a penis of a patient is inserted, and the drug outflow preventing device includes an opening that is formed extended from a portion where a glans of the patient is located.

## Description

### [Technical Field]

The present invention relates to a drug outflow preventing device.

### [Background Art]

Urethrostenosis is a symptom that a patient feels uncomfortable as a urethra becomes narrow by a scar, etc. which appears during a healing process of a wound created in a urethral mucosa because of an inflammation or an injury. An inside of a urethra becomes narrow as stenosis occurs in a urethra of a penis of a patient. In case of a patient having urethrostenosis, a surgery is operated with an endoscope to make a stenosed urethra wide.

Meanwhile, the biggest problem of treating urethrostenosis is that a relapse rate is over 50-70% due to fibrotic reaction after a surgery. Therefore, to suppress a relapse by restenosis after a surgery, various and expensive drugs for preventing stenosis are used.

However, for administering a drug to prevent a relapse of stenosis, a primitive method is used that a drug is administered beside a catheter, and a gauze is tied in a catheter, etc. to keep a drug from leaking outside a body.

Therefore, to treat urethrostenosis, an expensive drug has to be administered repeatedly for multiple times. Accordingly, an expensive drug may be wasted. Also, a patient has to be still for a day because a catheter is fixed to abdominal region to keep a drug in a urethra from leaking outside a body, which is a problem. A patient also feels uncomfortable due to a primitive method of tiding a gauze in a catheter.

### [Disclosure]

### [Technical Problem]

The present invention is to solve the above problem and to provide a drug outflow preventing device to prevent an outflow of a drug, by effectively sealing glans urethra area.

A targeted problem of the disclosure is not limited by the problems which are mentioned above, and other problems may be understood by a person skilled in the relevant field of technology, from the following description.

### [Solution to Problem]

According to an embodiment of the present invention, a drug outflow preventing device to solve the above problem is a drug outflow preventing device into which a penis of a patient is inserted, and the drug outflow preventing device includes an opening that is formed extended from a portion where a glans of the patient is located.

Also, in the opening, an inner diameter of one end extended from a portion where a glans of the patient is located may be formed smaller than an inner diameter of the other end.

Also, the opening may have a projection protruded toward an internal hollow.

Also, the projection may be formed to be closer to a portion where a glans of the patient is located.

Also, the projection may be formed to be leaned toward a urethra of the patient.

Also, in the drug outflow preventing device, a gel may be applied to a portion where a glans of the patient is located and the opening.

Also, a gelfoam formed by a gel may be further included, and the gelfoam may have a penetration hole a medical device penetrates that is inserted into a urethra of the patient.

Also, in the drug outflow preventing device, a portion where a glans of the patient is located and an opposite end of the opening may be formed thicker.

Also, in the drug outflow preventing device, a thickness of the opening may be formed over 3 times thicker than a thickness of a portion into which a penis of the patient is inserted.

Other features and aspects will be apparent from the following detailed description, the drawings, and the claims.

### [Effects of Invention]

According to the present invention, a high sealing force may be maintained so that a drug is not leaked around a urethra of a patient into which a catheter, etc. is inserted.

Also, with reducing discomfort when applying a device, it may be fixed well to an external genitalia.

### [Description of Drawings]

FIG. 1 is a cross-sectional view of a drug outflow preventing device according to a first embodiment of the present invention.
FIG. 2 is a perspective view of a drug outflow preventing device according to a first embodiment of the present invention.
FIG. 3 is a cross-sectional view of a drug outflow preventing device according to a second embodiment of the present invention.
FIG. 4 is a cross-sectional view of a drug outflow preventing device according to a third embodiment of the present invention.
FIG. 5 illustrates a projection of an opening of a drug outflow preventing device.
FIG. 6 is a cross-sectional view of a drug outflow preventing device where a gel is applied to an area where a side of a glans is located.
FIG. 7 is a cross-sectional view of a drug outflow preventing device where a gel is applied to an opening and an area where a side of a glans is located.
FIG. 8 is a perspective view of a gelfoam where a catheter is inserted.
FIG. 9 is a cross-sectional view of a gelfoam having a penetration hole.
FIG. 10 is a cross-sectional view of a drug outflow preventing device according to a fourth embodiment of the present invention.

### [Best mode for Implementation of the Invention]

A drug outflow preventing device of the present invention is a drug outflow preventing device into which a penis of a patient is inserted, and the drug outflow preventing device includes an opening that is formed extended from a portion where a glans of the patient is located.

### [Method for Carrying out the Invention]

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the present invention of this application. For example, the sequences of operations described herein are merely examples, and are not limited to those set forth herein, but may be changed as will be apparent after an understanding of the present invention of this application, with the exception of operations necessarily occurring in a certain order. Also, descriptions of features that are known in the art may be omitted for increased clarity and conciseness.

The features described herein may be embodied in different forms, and are not to be construed as being limited to the examples described herein. Rather, the examples described herein have been provided merely to illustrate some of the many possible ways of implementing the methods, apparatuses, and/or systems described herein that will be apparent after an understanding of the present invention of this application.

Although the first, second, etc. are used to describe various elements, components and / or sections, it is needless to say that these elements, components and / or sections are not limited by these terms. These terms are only used to distinguish one element, element or section from another element, element or section. Therefore, it goes without saying that the first element, the first element or the first section mentioned below may be the second element, the second element or the second section within the technical spirit of the present invention.

The terminology used herein is for the purpose of illustrating embodiments and is not intended to be limiting of the present invention. In the present specification, the singular form includes plural forms unless otherwise specified in the specification. It is noted that the terms "comprises" and / or "comprising" used in the specification are intended to be inclusive in a manner similar to the components, steps, operations, and / Or additions.

Unless defined otherwise, all terms (including technical and scientific terms) used herein may be used in a sense commonly understood by one of ordinary skill in the art to which this invention belongs. Also, commonly used predefined terms are not ideally or excessively interpreted unless explicitly defined otherwise.

A detailed description is given below, with attached drawings.

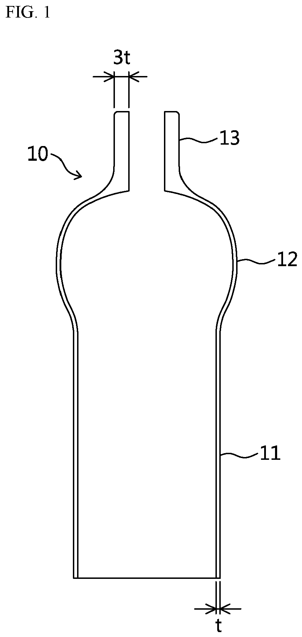

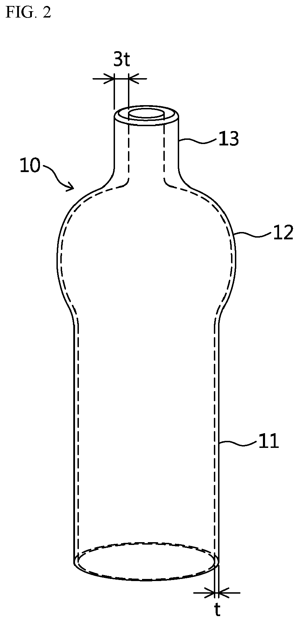

FIG. 1 is a cross-sectional view of a drug outflow preventing device according to a first embodiment of the present invention, and FIG. 2 is a perspective view of a drug outflow preventing device according to a first embodiment of the present invention.

Referring to FIG. 1 and FIG. 2, one side of a drug outflow preventing device 10 according to a first embodiment of the present invention is opened, and a penis of a patient is inserted. Also, it includes an opening 13 that is formed extended from a portion where a glans of a patient is located, which is the opposite side of an opened side.

Specifically, a drug outflow preventing device 10 may be divided into 3 parts. A body unit 11 into which a penis of a patient is inserted, a connection unit 12 to connect a gap between the body unit 11 and an opening 13, and an opening 13 formed to be extended from the connection unit 12. An external genitalia of a patient is located in a body unit 11 and a connection unit 12.

Major portions of an external genitalia of a patient are located in a body unit 11, and its one side may be opened, and it may have a cylindrical shape. A body unit 11 is longer than an opening 13, and a diameter of its internal cross section is large.

A connection unit 12 covers a glans, and it may have a convex shape or bowl shape to connect a gap between a body unit 11 and an opening 13. It may be desirable that a connection unit 12 is adequately formed to fit into a form of a glans. Through that, it may be easy to wear a device on a glans, and it may adhere to a glans well. A thickness of a connection unit 12 may be formed to be identical with a thickness of a body unit 11.

An opening 13 is formed to be extended from a connection unit 12, and it plays a role as a guide-hole to guide by inserting a catheter, etc. That is, an opening 13 is formed to be extended from a portion where a glans of a patient is located.

When a catheter, etc. is inserted into the opening 13, it may adhere and be fixed to a catheter by elasticity of an opening 13.

In this case, to maximize an effect to adhere and be fixed with inserting a medical device such as a catheter, an internal diameter of an opening 13 toward a urethra may be formed to be smaller than an internal diameter of an end, and an opening 13 may be formed to be thicker than a body unit 11.

Referring to FIG. 1 and FIG. 2 again, it is shown that a thickness of an opening 13 is 3 times thicker than a thickness of a body unit 11, and desirably, it may be over 3 times thicker. Hereupon, it makes it easy to insert a medical device such as a catheter, which is inserted into a urethra of a patient, and adhering characteristic of a medical device inserted into a urethra of a patient may be improved and supported.

A drug outflow preventing device 10 may be composed of at least one of silicon, rubber, latex, or urethane. Since they have excellent elasticity and durability, it may enhance convenience of installing and using. That is, as a drug outflow preventing device 10 is formed by an elastic material such as silicon, rubber, latex, urethane, etc., it may feel soft and have enough space when a penis is enlarged and shrunk.

The drug outflow preventing device 10 may be rolled up before it is used. It is to prevent any damage of a drug outflow preventing device 10 and to reduce a size of a drug outflow preventing device 10.

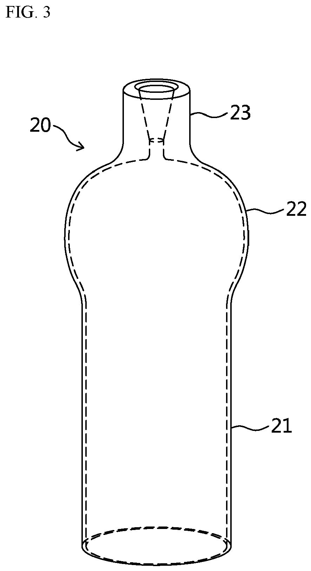

FIG. 3 is a cross-sectional view of a drug outflow preventing device according to a second embodiment of the present invention.

Referring to FIG. 3, in a drug outflow preventing device 20 according to a second embodiment of the present invention, a shape of an opening 23 is changed from a drug outflow preventing device 10 according to a first embodiment of the present invention. Other structures are identical, and therefore, a detailed description is omitted.

In FIG. 3, a cross-sectional diameter of an internal space in an opening 23 is reduced, when it is getting closer to a urethra. Specifically, an opening 23 may be formed with a slope. When a catheter is inserted toward a urethra, a diameter of an internal space in an opening 23 is reduced more and more, resulting in making it easy to insert a catheter and preventing a drug from leaking outside along an outer peripheral surface of a catheter, as a hole becomes narrow that is created by meeting an opening 23 and a connection unit 22.

Also, although it is not shown in a drawing, an inside of an opening 23 may be formed to be stepped, with having a stepped structure of 3 levels, 4 levels, etc. That is, there may be at least one or more portions in which an internal diameter becomes smaller, when moving from an entrance of an opening 23 toward a connection unit 22.

Accordingly, an inner diameter of one end extended from a portion where a glans of a patient is located may be formed smaller than an inner diameter of the other end, and adhering characteristic of a medical device such as a catheter that is inserted into a urethra of a patient may be improved, with preventing a leakage of a drug injected in a urethra.

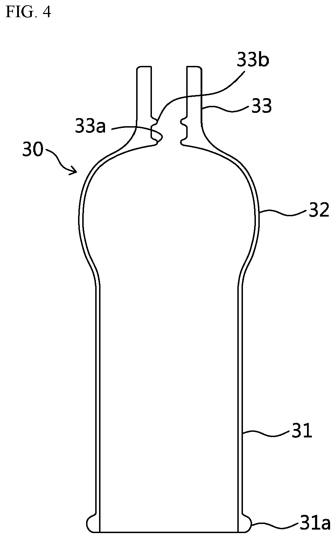

FIG. 4 is a cross-sectional view of a drug outflow preventing device according to a third embodiment of the present invention.

Referring to FIG. 4, a drug outflow preventing device 30 according to a third embodiment of the present invention includes an opening 33 that is formed extended from a portion where a glans of a patient is located. The opening 33 may have a projection protruded toward an internal hollow.

Specifically, an inlet jaw 33a may be formed in an opening 33 that is extended from a connection unit 32, and through making the innermost diameter which meets a glans small by the inlet jaw 33a, it may be possible to insert a medical device such as a catheter, etc., and it may adhere and be fixed to an inserted medical device such as a catheter, etc.

Also, at least one or more projections 33b may be formed in an internal hollow of an opening 33, and it may be possible to prevent a drug from leaking outside as much as possible by the projection 33b. In FIG. 4, based on a horizontally cut cross section, a projection 33b may be formed only in a partial area of an internal hollow, or it may be formed in an entire area like a ring shape. When a projection 33b is formed like a ring shape, an effect of preventing a leakage of a drug may be more enhanced, and it may play a role of an internal ring that is able to prevent a slip of an inserted medical device such as a catheter, etc.

In this case, a projection 33b may be formed to be closer to a portion where a glans of a patient is located. Since an opening 33 guides a urethra insertion of a medical device such as a catheter, a space may be provided for an apex of a medical device to be easily inserted into an opening 33.

Also, in a drug outflow preventing device 30 of FIG. 4, a portion where a glans of a patient is located and an opposite end of an opening may be formed to be thicker. That is, an end 31a of a body unit 31 may be formed thicker than other portions of a body unit 31.

By forming an end of a body unit 31 to be thicker than other portions of a body unit 31, it may be easy to roll and unroll a drug outflow preventing device 30. That is, it may be possible to effectively roll and unroll a body unit 31.

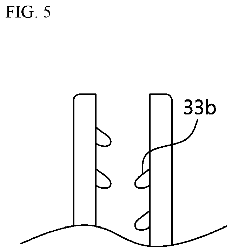

FIG. 5 illustrates a projection of an opening of a drug outflow preventing device.

Referring to FIG. 5, a projection 33b located in a middle of an opening may have a ring shape described above, and an upper projection and a lower projection may have a shape like a portion of a ring is split.

Specifically, to prevent a leakage of a drug and a slip, a ring-shaped projection 33b may be formed, and a projection having a split ring shape may be formed to make it easy to insert and take away a catheter.

Desirably, at least 3 projections 33b may be placed. A projection 33b located in a middle may have a ring shape, and an upper projection and a lower projection may have a shape like a portion of a ring is split. Specifically, an upper projection and a lower projection may have a ring shape split in half, and they may be symmetrically formed. When an upper projection and a lower projection are put together, a ring shape may appear.

Also, a projection 33b may be formed to be leaned toward a urethra of a patient. Accordingly, it may be easy to insert a medical device into a urethra, and it may be fundamentally prevented that an inserted medical device is moved toward an outside of a urethra.

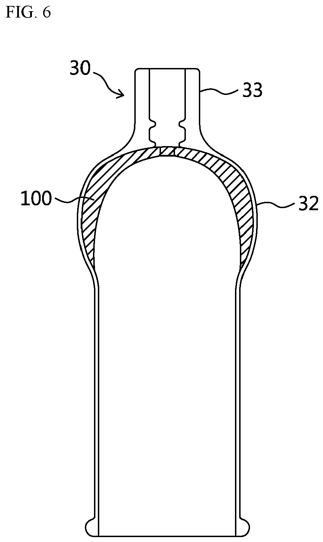

FIG. 6 is a cross-sectional view of a drug outflow preventing device where a gel is applied to an area where a side of a glans is located.

Referring to FIG. 6, in a drug outflow preventing device 30 according to a third embodiment, a gel may be applied to a partial area of a connection unit 31. That is, a drug outflow preventing device 30 may apply a gel to a portion where a glans of a patient is located. Of course, a gel may be applied to a portion where a glans of a patient is located, not only in a first drug outflow preventing device 10 and a second drug outflow preventing device 20, but also in another form of a drug outflow preventing device that is easily changeable from the present invention.

A shape may be made by a gel, and it is referred to a gelfoam. That is, a gelfoam 100 is composed of a gel, and it may be composed of a soft silicone gel or a hydro gel to relieve discomfort when wearing a drug outflow preventing device and to make it fixed well to a side of a glans. Also, a high sealing force may be maintained due to characteristics of a gel so that a drug is not leaked around a urethra where a catheter is inserted.

That is, by applying a gelfoam 100 to a glans that is sensitive to a sense or a pain, a leakage of drug from a urethra to an outside may be efficiently prevented as a sealing force is maintained by characteristics of a gelfoam 100 around an entrance of a urethra. Especially, by applying a gelfoam 100 having a slight viscosity to a side of a glans, a pain and discomfort may be relieved, and an excellent fixing force may be given to an external genitalia.

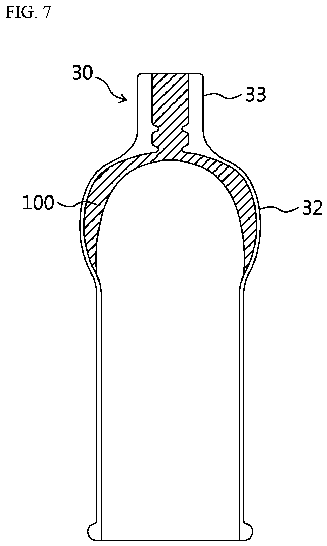

FIG. 7 is a cross-sectional view of a drug outflow preventing device where a gel is applied to an opening and an area where a side of a glans is located. Referring to FIG. 7, in a drug outflow preventing device 30 according to a third embodiment, a gel may be applied to a partial area of a connection unit 32 and an opening 33. That is, a gel may be applied up to an opening 33 besides a portion where a glans of a patient is located. Of course, a gel may be applied to a portion where a glans of a patient is located and an opening, not only in a first drug outflow preventing device 10 and a second drug outflow preventing device 20, but also in another form of a drug outflow preventing device that is easily changeable from the present invention.

By a gelfoam 100 that is composed of a gel which is fully filled in an opening 33, a leakage of a drug may be fundamentally prevented as a completely sealed state is maintained.

Also, a catheter may be inserted into a gelfoam 100 that is located in an opening, and a penetrated catheter may maintain a completely sealed state by characteristics of a gel.

In this case, an opening 33 may not be always opened. A penetration hole may be formed as a catheter is inserted by a force inserting a medical device such as a catheter, and when a medical device is removed, an empty space of an opening 33 may be filled with a gel by viscosity of a gel.

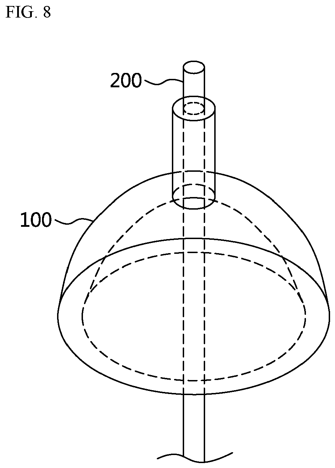

FIG. 8 is a perspective view of a gelfoam where a catheter is inserted.

Referring to FIG. 8, a gelfoam 100 may have a bowl shape to which a stick is connected.

Although a catheter 200 is inserted into a middle of a stick, a complete sealing state without an empty space may be maintained by a gel and its viscosity. By a gelfoam 100 that is located in a connection unit of a device, a patient may feel softness in a glans, and wearability of a drug outflow preventing device may be improved.

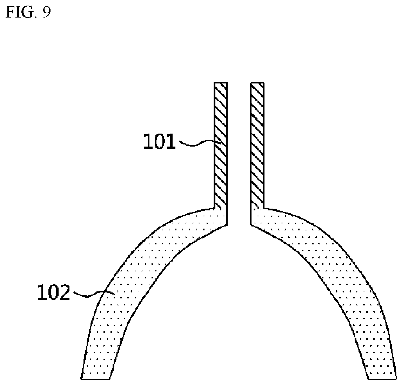

FIG. 9 is a cross-sectional view of a gelfoam having a penetration hole.

Referring to FIG. 9, a gelfoam may have a penetration hole that is penetrated by a medical device inserted into a urethra of a patient. By characteristics of a gel, a penetration hole may be only formed when a medical device such as a catheter, etc. is inserted, but a penetration hole may be maintained at all times.

As shown in FIG. 9, when a gelfoam always has a penetration hole, there may be difference in components of a gel between a gelfoam 102 with a bowl shape where a side of a glans of a drug outflow preventing device is located and a gelfoam 101 located in an opening that is formed extended from a portion where a glans of a patient is located. In this case, components of a gel of a gelfoam 101 located in an opening may have a higher viscosity.

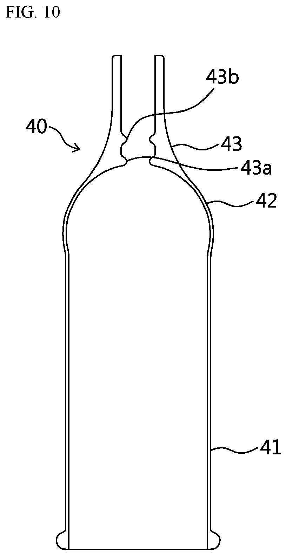

FIG. 10 is a cross-sectional view of a drug outflow preventing device according to a fourth embodiment of the present invention.

Referring to FIG. 10, in a drug outflow preventing device 40 according to a fourth embodiment of the present invention, a connection unit 42 covering a glans may be formed wider than a drug outflow preventing device 30 shown in FIG. 4 in terms of an angle connected from an opening 43 to a body unit 41. Accordingly, it is easy to wear a device to a glans, and adhering may be improved.

Meanwhile, although it is not illustrated in drawings, a material for wound dressing (not shown) located inside may be further included in drug outflow preventing devices 10, 20, 30, 40 described above and another form of a drug outflow preventing device that is easily changeable from drug outflow preventing devices 10, 20, 30, 40 of the present invention.

After a surgery of a penis, a dressing has to be cut and used to fit into a shape of a penis due to a specific shape of a penis. To improve the discomfort, it may be desirable to apply and use a wound dressing inside drug outflow preventing devices 10, 20, 30, 40 in advance. Especially, a wound dressing may be composed of a portion that fits into a side of a glans and another portion that fits into a side of a penis under a side of a glans, and it may be installed inside drug outflow preventing devices 10, 20, 30, 40.

According to drug outflow preventing devices of the present invention, a leakage of a drug may be efficiently prevented, and it may be easy to insert a medical device such as a catheter. Also, an inserted catheter may be remarkably fixed.

The above-described exemplary embodiments are examples for describing the present invention, but the present invention is not limited thereto. The present invention may be carried out in various forms by those skilled in the art so that a technical scope of the present invention should be defined by the accompanying claims.

### [Industrial Applicability]

The present invention can be used to treat urological disease.

## Claims

1. A drug outflow preventing device into which a penis of a patient is inserted,
wherein the drug outflow preventing device includes an opening that is formed extended from a portion where a glans of the patient is located.

2. The drug outflow preventing device of Claim 1,
wherein an inner diameter of one end extended from a portion where a glans of the patient is located is formed smaller than an inner diameter of the other end in the opening.

3. The drug outflow preventing device of Claim 1,
wherein the opening has a projection protruded toward an internal hollow.

4. The drug outflow preventing device of Claim 3,
wherein the projection is formed to be closer to a portion where a glans of the patient is located.

5. The drug outflow preventing device of Claim 3,
wherein the projection is formed to be leaned toward a urethra of the patient.

6. The drug outflow preventing device of Claim 1,
wherein a gel is applied to a portion where a glans of the patient is located and the opening, in the drug outflow preventing device.

7. The drug outflow preventing device of Claim 1,
wherein a gelfoam formed by a gel is further included,
wherein the gelfoam has a penetration hole a medical device penetrates that is inserted into a urethra of the patient.

8. The drug outflow preventing device of Claim 1,
wherein a portion where a glans of the patient is located and an opposite end of the opening are formed thicker, in the drug outflow preventing device.

9. The drug outflow preventing device of Claim 1,
wherein a thickness of the opening is formed over 3 times thicker than a thickness of a portion into which a penis of the patient is inserted, in the drug outflow preventing device.
